# EUROPEAN PATENT APPLICATION

(11) **EP 4 467 021 A1**
(43) Date of publication of application: **27.11.2024**
(21) Application number: 23773848.9
(22) Date of filing: 21.03.2023
(51) Int. Cl.: A24F 40/46, A24F 40/40

(54) **ATOMIZER AND ELECTRONIC ATOMIZATION DEVICE**

(30) Priority: 23.03.2022 CN 202210292294
(71) Applicant: Shenzhen First Union Technology Co., Ltd., Shenzhen, Guangdong 518000 (CN)
(72) Inventor: XIE, Baofeng, Shenzhen, Guangdong 518000 (CN); HU, Ruilong, Shenzhen, Guangdong 518000 (CN); LEI, Baoling, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2023/082759
(87) International publication number: WO 2023/179601

(57) **Abstract**

An atomizer (100) and an electronic atomization device, the atomizer (100) being configured to atomize a liquid matrix to generate an aerosol and comprising: a housing (10), which defines a liquid storage cavity (12) for storing the liquid; a support (20), which is provided in the housing (10) and comprises a liquid channel (21), an accommodating groove (22) and a ventilation groove (23) recessed from a part of an inner side surface of the accommodating groove (22), the liquid channel (21) being in communication with the liquid storage cavity (12) and the accommodating groove (22); a capillary element (30), which is provided in the accommodating groove (22) and used for receiving and storing the liquid matrix from the liquid storage cavity (12) by means of the liquid channel (21); and a heating element (40), which is integrated on the surface of the capillary element (30), at least a part of the heating element(40) being exposed in the ventilation groove (23) such that the aerosol generated by the heating element (40) heating the liquid matrix can be released into the ventilation groove (23). The atomizer (100) has high atomization efficiency.

## Description

### CROSS-REFERENCE TO RELATED APPLICATIONS

This application claims priority to Chinese Patent Application No. 202210292294.4, filed with the China National Intellectual Property Administration on March 23, 2022 and entitled "ATOMIZER AND ELECTRONIC ATOMIZATION DEVICE", which is incorporated herein by reference in its entirety.

### TECHNICAL FIELD

The present invention relates to the field of electronic atomization technologies, and in particular, to an atomizer and an electronic atomization device.

### BACKGROUND

An electronic atomization device is an electronic product that heats a liquid substrate such as e-liquid or medicinal liquid and atomizes the liquid substrate into aerosols for inhalation.

The electronic atomization device may include an atomizer and a power supply assembly. The power supply assembly is configured to supply power to the atomizer. The atomizer may include an atomization core assembly and an atomization chamber. The atomization core assembly is configured to generate heat when powered on to atomize a liquid substrate, and the atomization chamber is configured to supply the atomization core assembly with a to-be-heated and atomized liquid substrate.

The atomizer usually uses a porous ceramic body, liquid guide cotton, and the like as a capillary element for absorbing the liquid substrate, and heats at least a part of the liquid substrate in the capillary element by using a heating element disposed in contact with an atomization surface of the capillary element to generate aerosols.

In a related atomizer structure, the heating element is usually disposed on only one surface of the capillary element, for example, an upper surface or a lower surface. As a result, the atomization efficiency is low. In addition, the related atomizer includes a large quantity of structures, and a mounting structure and mounting manner are also complicated.

### SUMMARY

The present invention provides an atomizer and an electronic atomization device having the atomizer, to resolve a technical problem that the atomization efficiency of a current atomizer is low.

To resolve the technical problem, the following technical solutions in the present invention are used: an atomizer, configured to atomize a liquid substrate to generate aerosols. The atomizer includes: a housing, defining a liquid storage cavity for storing the liquid substrate; a frame, disposed in the housing and including a liquid channel, an accommodating groove, and a vent groove recessed from a part of an inner side surface of the accommodating groove, where the liquid channel is communicated with the liquid storage cavity and the accommodating groove; a capillary element, disposed in the accommodating groove and configured to receive and store the liquid substrate from the liquid storage cavity through the liquid channel; and a heating element, bond to a surface of the capillary element, where at least a part of the heating element is exposed in the vent groove, so that the aerosols generated by the heating element heating the liquid substrate can be released into the vent groove.

In a preferred implementation, the vent groove includes a first vent groove and a second vent groove arranged opposite to each other, there are two heating elements, at least a part of one heating element is exposed in the first vent groove, and at least a part of the other heating element is exposed in the second vent groove.

In a preferred implementation, the capillary element has two side surfaces facing the first vent groove and the second vent groove, and the two heating elements are respectively bond to different side surfaces.

In a preferred implementation, the housing further includes a smoke exhaust tube, the frame further includes an insertion hole and a communication groove communicated with the insertion hole, an end of the smoke exhaust tube is inserted into the insertion hole, and the communication groove is communicated with the vent groove and the smoke exhaust tube.

In a preferred implementation, a plurality of liquid guide grooves are provided on a channel wall of the liquid channel, and the liquid guide grooves extend by the entire length of the liquid channel; and/or liquid guide cotton is provided in the liquid channel, and the liquid guide cotton extends by at least a part of the length of the liquid channel.

In a preferred implementation, at least one ventilation groove is provided on a side groove wall of the accommodating groove, and the ventilation groove is configured to be communicated with external atmosphere and the liquid channel.

In a preferred implementation, the frame is made of a flexible material, and at least a part of the frame abuts against the surface of the capillary element and forms a seal between the liquid channel and the vent groove to prevent the liquid substrate in the liquid channel from directly flowing into the vent groove.

In a preferred implementation, the capillary element further includes an upper surface, a lower surface, and two side surfaces extending between the upper surface and the lower surface. The heating element includes a first connection end, a second connection end, and a heating portion bending and extending between the first connection end and the second connection end. In addition, two first connection ends of the two heating elements are connected via a first connection portion disposed on the lower surface, and two second connection ends of the two heating elements are connected via a second connection portion disposed on the lower surface.

In a preferred implementation, the atomizer further includes a base. The base is connected to the housing and is configured to hold the frame in the housing.

In a preferred implementation, the frame includes an annular surrounding portion, and the vent groove extends to an inner side of the annular surrounding portion. The base includes an annular insertion portion, and a top end of the annular insertion portion is provided with two recesses. In addition, the annular insertion portion is inserted in the annular surrounding portion, and the two recesses are communicated with the two vent grooves respectively.

In a preferred implementation, the atomizer further includes two electrode columns. The two electrode columns are mounted on the base and respectively abut against the first connection portion and the second connection portion disposed on the lower surface of the capillary element. The first connection portion and the second connection portion are connected to two ends of the heating elements respectively.

To resolve the technical problem, the following technical solutions in the present invention are further used: an electronic atomization device, including an atomizer atomizing a liquid substrate to generate aerosols, and a power supply assembly supplying power to the atomizer. The atomizer includes any one of the foregoing atomizer.

The present invention has the following beneficial effects: in the atomizer of this embodiment, the inner side surface of the accommodating groove of the frame is partially recessed to form the vent groove, and the heating element is disposed on the side surface of the capillary element, so that when the capillary element and the heating element are disposed in the accommodating groove and the heating element is powered on, at least a part of the heating element may be exposed in the vent groove, and the heating element may heat and atomize a liquid substrate on the side surface, and aerosols formed by atomization are transported into the smoke exhaust tube through the vent groove. Accordingly, the atomizer of this embodiment and the electronic atomization device using the atomizer have high atomization efficiency.

### BRIEF DESCRIPTION OF THE DRAWINGS

One or more embodiments are exemplarily described with reference to the corresponding accompanying drawings, and the descriptions are not to be construed as limiting embodiments. Elements in the accompanying drawings that have same reference numerals are represented as similar elements, and unless otherwise particularly stated, the figures in the accompanying drawings are not drawn to scale.
FIG. 1 is a schematic diagram of three-dimensional assembly of an atomizer according to an embodiment of the present invention;
FIG. 2 is a schematic diagram of other three-dimensional assembly of the atomizer shown in FIG. 1;
FIG. 3 is a schematic cross-sectional view of the atomizer shown in FIG. 1;
FIG. 4 is another schematic cross-sectional view of the atomizer shown in FIG. 1;
FIG. 5 is a schematic exploded view of the atomizer shown in FIG. 1;
FIG. 6 is a three-dimensional schematic diagram of a housing of the atomizer shown in FIG. 5;
FIG. 7 is a three-dimensional schematic diagram of a frame of the atomizer shown in FIG. 5;
FIG. 8 is a three-dimensional schematic diagram of a capillary element and a heating element of the atomizer shown in FIG. 5; and
FIG. 9 is a three-dimensional schematic diagram of a base of the atomizer shown in FIG. 5.

### DETAILED DESCRIPTION

For ease of understanding the present invention, the present invention is described in more detail below with reference to the accompanying drawings and specific embodiments. It should be noted that when an element is expressed as "being fixed to" another element, the element may be directly on the another element, or one or more intermediate elements may exist between the element and the another element. When one component is expressed as "being connected to" another component, the component may be directly connected to the another component, or one or more intermediate components may exist between the component and the another component. The terms "vertical", "horizontal", "left", "right", "inner", "outside", and similar expressions used in this specification are merely used for an illustrative purpose.

Unless otherwise defined, meanings of all technical and scientific terms used in the specification are the same as that usually understood by a person skilled in the art to which the present invention belongs. Terms used in the specification of the present invention are merely intended to describe objectives of the specific embodiment, and are not intended to limit the present invention. The term "and/or" used in this specification includes any or all combinations of one or more related listed items.

In addition, technical features involved in different embodiments of the present invention described below may be combined together if there is no conflict.

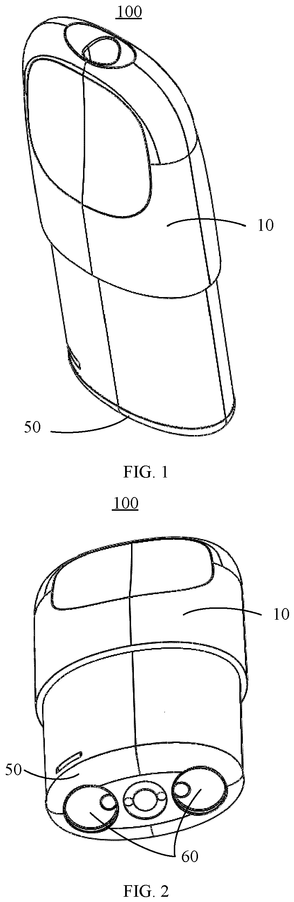

FIG. 1 to FIG. 5 are respectively a schematic diagram of three-dimensional assembly, a schematic cross-sectional view, and a schematic exploded view of an atomizer 100 according to an embodiment of the present invention. The atomizer 100 is configured to atomize a liquid substrate to generate aerosols, and may include a housing 10, a frame 20, a capillary element 30, a heating element 40, a base 50, and an electrode column 60. The housing 10 and the base 50 may be mounted in a fitting manner to form interior space, and then the frame 20, the heating element 40, and the capillary element 30 are mounted and accommodated in the interior space.

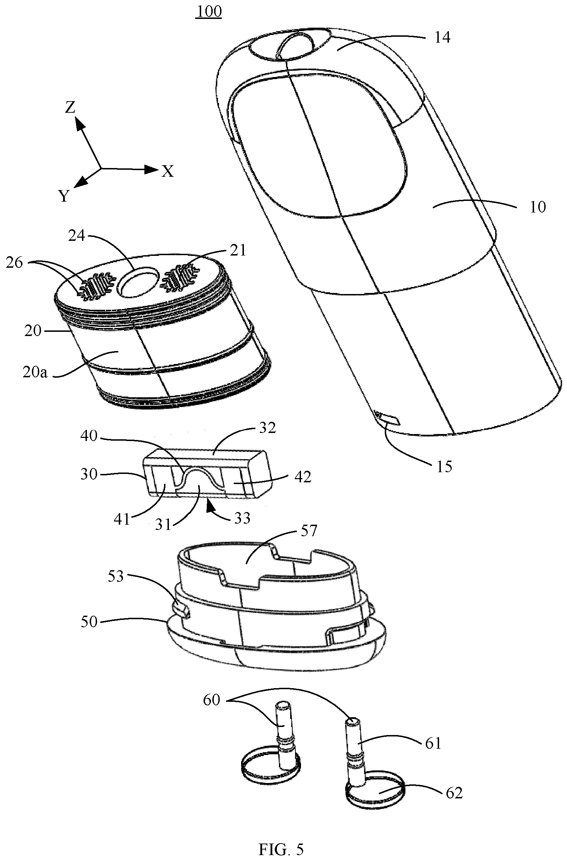

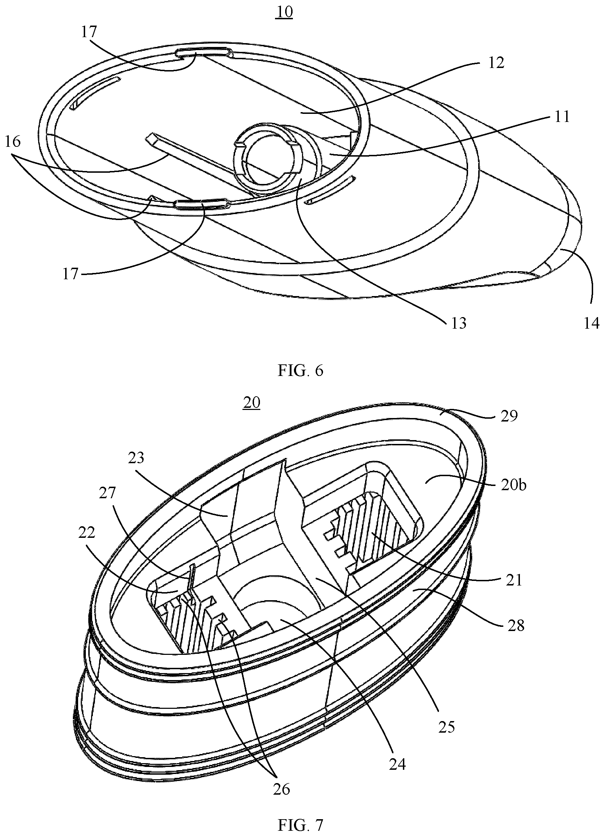

FIG. 6 is a three-dimensional schematic diagram of the housing 10 of the atomizer 100 shown in FIG. 5. The housing 10 includes a smoke exhaust tube 11 and defines a liquid storage cavity 12 for storing the liquid substrate. The liquid storage cavity 12 is accommodation space defined in the housing 10 and is configured to store the liquid substrate and supply the liquid substrate to the capillary element 30. The liquid storage cavity 12 may be further sealed by the frame 20. For example, the liquid substrate may be liquid such as e-liquid or medicinal liquid. In this specification, the liquid substrate may also be referred to as liquid, atomization may also be referred to as vaporization, and the aerosol may also be referred to as smoke, mist, or atomized gas. The housing 10 may further provide a mouthpiece portion 14 connected to the smoke exhaust tube 11. The smoke exhaust tube 11 defines a hollow tube for allowing aerosols formed on an atomization surface of the capillary element 30 to pass through and be discharged through the mouthpiece portion 14.

FIG. 7 is a three-dimensional schematic diagram of the frame 20 of the atomizer 100 shown in FIG. 5. The frame 20 is disposed in the housing 10 and includes a liquid channel 21, an accommodating groove 22, and a vent groove 23 recessed from a part of an inner side surface of the accommodating groove 22. The liquid channel 21 is communicated with the liquid storage cavity 12 and the accommodating groove 22. There may be two liquid channels 21, and the two liquid channels 21 are distributed in an X direction in FIG. 5 on both sides of a central axis that is of the frame 20 and that is parallel to a Z direction. The frame 20 may include a frame main body 20a, and cross sections of the frame 20 in planes along the X direction and a Y direction in FIG. 5 are substantially elliptical. The liquid channel 21 may extend inwardly from a top end of the frame main body 20a along the Z direction until the liquid channel 21 is communicated with the accommodating groove 22. The accommodating groove 22 may be an elongated groove extending along the X direction, and is configured to be fitted to the capillary element 30 in a rectangular parallelepiped shape. The vent groove 23 may be provided on the frame main body 20a. In an embodiment, the frame 20 further includes an insertion hole 24 and a communication groove 25 communicated with the insertion hole 24. An end 13 of the smoke exhaust tube 11 is inserted into the insertion hole 24, and the communication groove 25 is communicated with the vent groove 23 and the smoke exhaust tube 11. In an embodiment shown in FIG. 7, both the communication groove 25 and the vent groove 23 are communicated with the accommodating groove 22, and the communication groove 25 and the accommodating groove 22 may be arranged at an upper position and a lower position, respectively. The frame 20 may be made of a flexible material such as silicone or thermoplastic elastomer (TPE), and may be inserted into the housing 10 to form a seal with the housing 10. In addition, at least a part of the frame 20 abuts against a surface of the capillary element 30 and forms a seal between the liquid channel 21 and the vent groove 23 to prevent the liquid substrate in the liquid channel 21 from directly flowing into the vent groove 23.

The capillary element 30 is disposed in the accommodating groove 22 and is configured to receive and store the liquid substrate from the liquid storage cavity 12 through the liquid channel 21. The capillary element 30 may be made of a material having a capillary channel or a pore, such as fiber wool, a porous ceramics body, a glass fiber rope, porous glass ceramics, porous glass, and another hard or rigid capillary structure. The capillary element 30 may be substantially in a rectangular shape, an upper surface 32 of the capillary element 30 is disposed to attach to a bottom of the accommodating groove 22 of the frame 20, so that the liquid channel 21 is covered. In this way, the liquid substrate stored in the liquid storage cavity 12 can be transported to the capillary element 30 through the liquid channel 21 without leaking to other surfaces of the capillary element 30 through the liquid channel 21.

The heating element 40 is bond to the surface of the capillary element 30. At least a part of the heating element 40 is exposed in the vent groove 23, so that the aerosols generated by the heating element 40 heating the liquid substrate can be released into the vent groove 23.

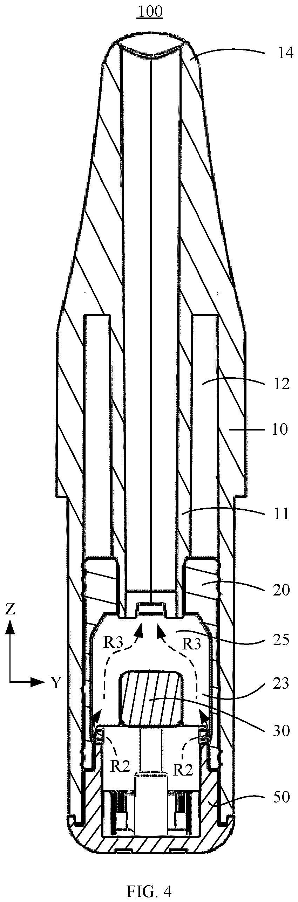

Further, the vent groove 23 may include a first vent groove and a second vent groove arranged opposite to each other, and there may be two heating elements 40. At least a part of one heating element 40 is exposed in the first vent groove, and at least a part of the other heating element 40 is exposed in the second vent groove. In addition, the capillary element 30 has two side surfaces 31 facing the first vent groove and the second vent groove, and the two heating elements 40 are respectively bond to different side surfaces 31. The two side surfaces 31 are disposed opposite to each other and are parallel to planes of the X direction and the Z direction. Therefore, when the heating element 40 is powered on, the heating element 40 may heat and atomize the liquid substrate on the side surface 31, and aerosols formed by atomization may, along two routes R3 shown in FIG. 4, pass through the vent groove 23 and the communication groove 25 in sequence and enter the smoke exhaust tube 11.

In the atomizer 100 of this embodiment, the inner side surface of the accommodating groove 22 of the frame 20 is partially recessed to form the vent groove 23, and the heating element 40 is disposed on the side surface 31 of the capillary element 30, so that when the capillary element 30 and the heating element 40 are disposed in the accommodating groove 22, and the heating element 40 is powered on, at least a part of the heating element 40 may be exposed in the vent groove 23, the heating element 40 may heat and atomize the liquid substrate on the side surface 31, and the aerosols formed by atomization are transported into the smoke exhaust tube 11 through the vent groove 23. Accordingly, the atomizer 100 of this embodiment has high atomization efficiency.

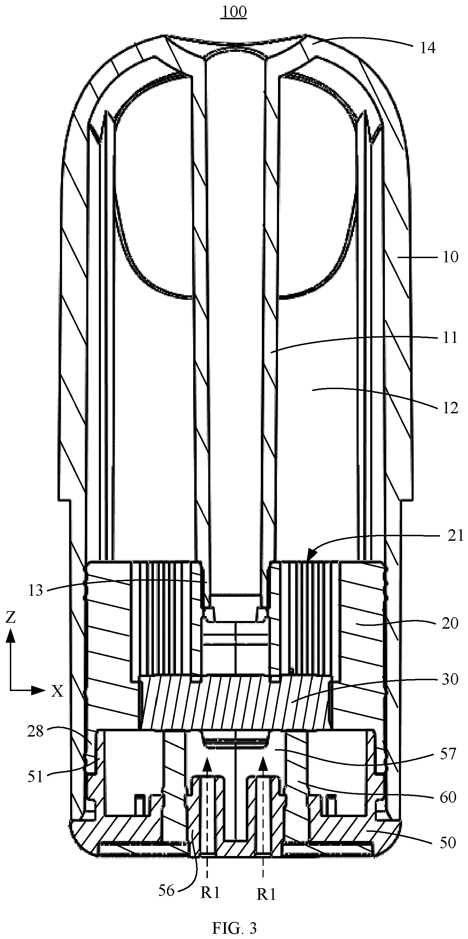

In an optional embodiment, with reference to FIG. 3, FIG. 5, and FIG. 7, a plurality of liquid guide grooves 26 are provided on a channel wall of the liquid channel 21, and the liquid guide grooves 26 extend by the entire length of the liquid channel 21. A cross-section of the liquid channel 21 may be circular, elliptical, square, or the like, and the plurality of liquid guide grooves 26 on the channel wall of the liquid channel 21 may be evenly distributed. The liquid guide groove 26 extends along the Z square by the same length as the liquid channel 21. The liquid guide groove 26 may be of a capillary structure. The plurality of liquid guide grooves 26 are provided, so that a liquid substrate such as e-liquid may be transported from the liquid storage cavity 12 to the capillary element 30 more easily.

In another optional embodiment, liquid guide cotton (not shown) may be provided in the liquid channel 21, and the liquid guide cotton extends by at least a part of the length of the liquid channel 21. Further, the liquid guide cotton may also be provided in the liquid channel 21 having the plurality of liquid guide grooves 26. The liquid guide cotton is provided, so that similarly, the liquid substrate may be transported from the liquid storage cavity 12 to the capillary element 30 more easily. The liquid guide cotton can absorb a part of the liquid substrate, so that the liquid guide cotton has a specific capability to retain liquid. Therefore, the liquid guide cotton can control a rate at which the liquid substrate is transported from the liquid storage cavity 12 to the capillary element 30, to prevent a part of the liquid substrate from leaking from between the capillary element 30 and the frame 20.

In an optional embodiment, with reference to FIG. 7, at least one ventilation groove 27 is provided on a side groove wall of the accommodating groove 22, and the ventilation groove 27 is configured to be communicated with external atmosphere and the liquid channel 21. When the base 50 is connected to the housing 10, the ventilation groove 27 may be communicated with interior space 57 of the base 50, and the interior space 57 of the base 50 is further communicated with the external atmosphere. The ventilation groove 27 may form a small-diameter channel such as a capillary channel with the capillary element 30 accommodated in the accommodating groove 22. When the ventilation groove 27 contains a liquid substrate, the liquid substrate and an absorption effect of the ventilation groove 27 can play a blocking role. Only when pressure in the liquid storage cavity 12 is lower than a specific level, a pressure difference between the external atmosphere and the liquid storage cavity 12 can cause outside air to flow into the liquid storage cavity 12 through the ventilation groove 27.

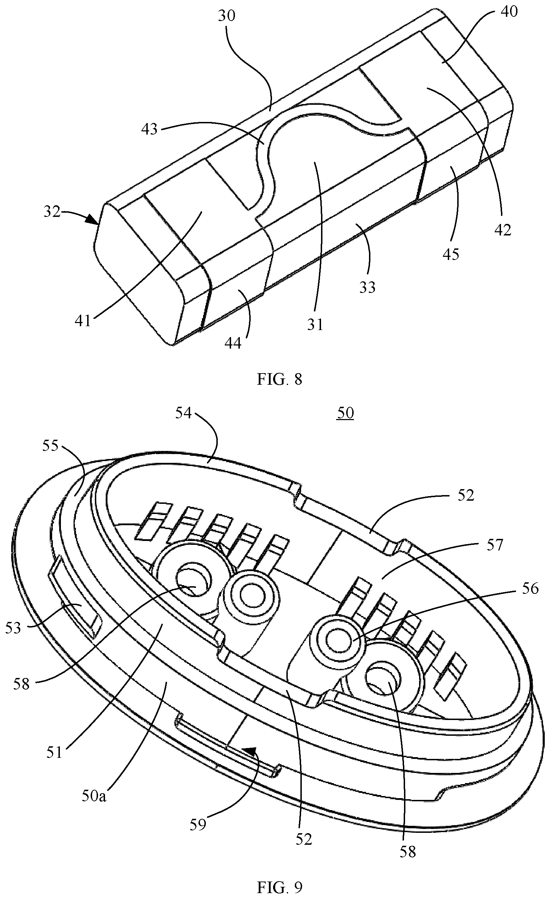

In an optional embodiment, with reference to FIG. 5 and FIG. 8, FIG. 8 is a three-dimensional schematic diagram of the capillary element 30 and the heating element 40 of the atomizer 100 shown in FIG. 5. The capillary element 30 further includes an upper surface 32 and a lower surface 33, and the two side surfaces 31 extend between the upper surface 32 and the lower surface 33. When the capillary element 30 is accommodated in the accommodating groove 22, the upper surface 32 may face the liquid channel 21, so that the liquid channel 21 is blocked by the upper surface 32, to cause the liquid substrate enters the capillary element 30 only from the upper surface 32 through internal pores.

The heating element 40 includes a first connection end 41, a second connection end 42, and a heating portion 43 bending and extending between the first connection end 41 and the second connection end 42. Two first connection ends 41 of the two heating elements 40 are connected via a first connection portion 44 disposed on the lower surface 33, and two second connection ends 42 of the two heating elements 40 are connected via a second connection portion 45 disposed on the lower surface 33. The heating element 40 and the first connection portion 44 and the second connection portion 45 may be integrally printed on the capillary element 30 by using a film printing technique. The first connection portion 44 and the second connection portion 45 are configured to be in conductive contact with the electrode column 60, so that a current flows through the heating portion 43. The heating portion 43 may be of a sheet-like structure with a plurality of holes, or may be of a circuit structure with a circuitous shape.

In an optional embodiment, with reference to FIG. 5 and FIG. 9, FIG. 9 is a three-dimensional schematic diagram of the base 50 of the atomizer 100 shown in FIG. 5. The base 50 may be connected to a slot 15 on the housing 10 in a snap-fit manner via, for example, a snap block 53, to hold the frame 20 in the housing 10. For example, with reference to FIG. 6, an inner wall of the housing 10 may be provided with a plurality of raised strips 16, and lower ends of the raised strips 16 away from the mouthpiece portion 14 are configured to abut against an upper end of the frame 20. With reference to FIG. 9, the base 50 includes an upper end surface 54 and a middle support surface 55. With reference to FIG. 7, the upper end surface 54 and the middle support surface 55 of the base 50 are configured to support a main body lower end surface 20b and a frame lower end surface 29 of the frame 20. The main body lower end surface 20b may be a lower end surface of the frame main body 20a, and the frame lower end surface 29 may be a lower end surface of the frame 20. In addition, the base 50 may include a slot 59, and an insertion block 17 may be further protruded from a lower end surface of the housing 10 away from the mouthpiece portion 14. During assembly, the insertion block 17 may be inserted into the slot 59. In this way, the frame 20 can be firmly held in the housing 10.

In an optional embodiment, with reference to FIG. 7, the frame 20 includes an annular surrounding portion 28, and the vent groove 23 extends to an inner side of the annular surrounding portion 28. The annular surrounding portion 28 may extend away from the mouthpiece portion 14 from the frame main body 20a in a direction. With reference to FIG. 9, the base 50 includes an annular insertion portion 51, and a top end of the annular insertion portion 51 is provided with two recesses 52. The annular insertion portion 51 may extend from a base body 50a of the base 50 toward the frame 20. An upper surface of the base body 50a forms the middle support surface 55, and the middle support surface 55 surrounds the annular insertion portion 51. The upper end surface 54 is recessed downwardly to form the recess 52. The base 50 may include one or more air inlet columns 56, the air inlet columns 56 has an airflow channel therein for guiding external air into the interior space 57 of the base 50.

With reference to FIG. 3, FIG. 4, FIG. 7, and FIG. 9, in an assembly structure, the annular insertion portion 51 is inserted in the annular surrounding portion 28, and the two recesses 52 are communicated with the two vent grooves 23 respectively. Therefore, when a user inhales from the mouthpiece portion 14 of the atomizer 100, air pressure in the atomizer 100 decreases, so that the external air enters the interior space 57 of the base 50 through the air inlet column 56 in a route R1 shown in FIG. 3. Then, air in the interior space 57 may enter the vent groove 23 through the recess 52 in a route R2 shown in FIG. 4. Accordingly, in the entire atomizer 100, the external air enters the vent groove 23 along the route R1 and the route R2 in sequence. In addition, when the heating element 40 is powered on, in the vent groove 23, the aerosols generated by the heating element 40 heating and atomizing the liquid substrate are carried into the smoke exhaust tube 11 along the route R3, and then the aerosols are discharged from the mouthpiece portion 14 for inhalation by the user.

In an optional embodiment, with reference to FIG. 3 and FIG. 5, there may be two electrode columns 60. The two electrode columns 60 are mounted on the base 50 and respectively abut against the first connection portion 44 and the second connection portion 45 disposed on the lower surface 33 of the capillary element 30. The first connection portion 44 and the second connection portion 45 are connected to two ends of the heating elements 40 respectively. The electrode column 60 may include a conductive post 61 and a conductive contact piece 62 connected thereto. The conductive post 61 extends from the conductive contact piece 62 toward the lower surface 33. The conductive contact piece 62 is exposed from the bottom of the base 50 and is configured to be electrically connected to a battery in a power supply assembly. With reference to FIG. 9, the base 50 may include a mounting hole 58 configured to allow the conductive post 61 to insert therein. The conductive post 61 may be interference-fitted to the mounting hole 58, or may be fitted to a top end of the mounting hole 58 via a snap ring on the conductive post 61 in a stopping manner.

In the atomizer 100 using the frame 20 made of a sealing silicone material, leakage and burning of the liquid substrate can be reduced. In addition, the capillary element 30 and the frame 20 are mounted in a fitting manner, so that complexity of the structure required for mounting a conventional ceramic core can be reduced. In addition, the atomizer 100 of this application has high utilization of space and e-liquid.

The various components of the atomizer 100 of the present invention are described above. In another embodiment of the present invention, an atomizer 100 and a power supply assembly may constitute an electronic atomization device. The atomizer 100 stores a liquid substrate and atomizes the liquid substrate to generate aerosols. The power supply assembly supplies power to the atomizer 100. When it is needed to inhale from the electronic atomization device having the atomizer 100, a power supply switch of the power supply assembly may be turned on first, so that a battery can supply power to a heating element 40 of the atomizer 100. Then, when a user inhales from a mouthpiece portion 14 of the atomizer 100, a controller in the electronic atomization device may enable the power supply assembly and the atomizer 100 to work based on an inhalation action, and finally, aerosols are generated for the user to inhale. A liquid substrate from a liquid storage cavity 12 is heated and atomized by the heating element 40 to form aerosols. External air may enter an interior space 57 of a base 50 through an air inlet column 56, and then enter a vent groove 23 through a recess 52, so that the external air is transported to an atomization surface of a capillary element 30 that contacts the heating element 40, and then formed aerosols are carried into a smoke exhaust tube 11 through a communication groove 25 and discharged through the mouthpiece portion 14.

Finally, it should be noted that: the foregoing embodiments are merely used for describing the technical solutions of the present invention, but are not intended to limit the present invention. Under the ideas of the present invention, the technical features in the foregoing embodiments or different embodiments may also be combined, the steps may be performed in any order, and many other changes of different aspects of the present invention also exists as described above, and these changes are not provided in detail for simplicity. Although the present invention is described in detail with reference to the foregoing embodiments, it should be appreciated by a person skilled in the art that modifications may still be made to the technical solutions described in the foregoing embodiments, or equivalent replacements may be made to the part of the technical features; and these modifications or replacements do not cause the essence of corresponding technical solutions to depart from the scope of the technical solutions in embodiments of the present invention.

## Claims

1. An atomizer, configured to atomize a liquid substrate to generate aerosols, wherein the atomizer comprises:
a housing, defining a liquid storage cavity for storing the liquid substrate;
a frame, disposed in the housing and comprising a liquid channel, an accommodating groove, and a vent groove recessed from a part of an inner side surface of the accommodating groove, wherein the liquid channel is communicated with the liquid storage cavity and the accommodating groove;
a capillary element, disposed in the accommodating groove and configured to receive and store the liquid substrate from the liquid storage cavity through the liquid channel; and
a heating element, bond to a surface of the capillary element, wherein at least a part of the heating element is exposed in the vent groove, so that the aerosols generated by the heating element heating the liquid substrate can be released into the vent groove.

2. The atomizer according to claim 1, wherein the vent groove comprises a first vent groove and a second vent groove arranged opposite to each other, the atomizer comprises two heating elements, at least a part of one heating element is exposed in the first vent groove, and at least a part of the other heating element is exposed in the second vent groove.

3. The atomizer according to claim 2, wherein the capillary element has two side surfaces facing the first vent groove and the second vent groove, and the two heating elements are respectively bond to different side surfaces.

4. The atomizer according to claim 1, wherein the housing further comprises a smoke exhaust tube, the frame further comprises an insertion hole and a communication groove communicated with the insertion hole, an end of the smoke exhaust tube is inserted into the insertion hole, and the communication groove is communicated with the vent groove and the smoke exhaust tube.

5. The atomizer according to claim 1, wherein:
a plurality of liquid guide grooves are provided on a channel wall of the liquid channel, and the liquid guide grooves extend by the entire length of the liquid channel; and/or
liquid guide cotton is provided in the liquid channel, and the liquid guide cotton extends by at least a part of the length of the liquid channel.

6. The atomizer according to claim 1, wherein at least one ventilation groove is provided on a side groove wall of the accommodating groove, and the ventilation groove is configured to communicate with external atmosphere and the liquid channel.

7. The atomizer according to claim 1, wherein the frame is made of a flexible material, and at least a part of the frame abuts against the surface of the capillary element and forms a seal between the liquid channel and the vent groove to prevent the liquid substrate in the liquid channel from directly flowing into the vent groove.

8. The atomizer according to claim 1, wherein:
the capillary element further comprises an upper surface, a lower surface, and two side surfaces extending between the upper surface and the lower surface, and the heating element comprises a first connection end, a second connection end, and a heating portion bending and extending between the first connection end and the second connection end; and
two first connection ends of the two heating elements are connected via a first connection portion disposed on the lower surface, and two second connection ends of the two heating elements are connected via a second connection portion disposed on the lower surface.

9. The atomizer according to any one of claims 1 to 8, wherein the atomizer further comprises a base, and the base is connected to the housing and is configured to hold the frame in the housing.

10. The atomizer according to claim 9, wherein:
the frame comprises an annular surrounding portion, and the vent groove extends to an inner side of the annular surrounding portion;
the base comprises an annular insertion portion, and a top end of the annular insertion portion is provided with two recesses; and
the annular insertion portion is inserted in the annular surrounding portion, and the two recesses are communicated with the two vent grooves respectively.

11. The atomizer according to claim 9, wherein:
the atomizer further comprises two electrode columns, the two electrode columns are mounted on the base and respectively abut against the first connection portion and the second connection portion disposed on the lower surface of the capillary element; and
the first connection portion and the second connection portion are connected to two ends of the heating elements respectively.

12. An electronic atomization device, comprising an atomizer atomizing a liquid substrate to generate aerosols, and a power supply assembly supplying power to the atomizer, wherein the atomizer comprises the atomizer according to any one of claims 1 to 11.
